# EUROPÄISCHE PATENTSCHRIFT

(11) **EP 0 840 117 B1**
(45) Veröffentlichungstag und Bekanntmachung des Hinweises auf die Patenterteilung: **30.05.2001**
(21) Anmeldenummer: 97117858.7
(22) Anmeldetag: 15.10.1997
(51) Int. Cl.: G01N 33/00

(54) **Verfahren und Vorrichtung zum Ermitteln der Konzentration eines Stoffes in einem gasförmigen Medium**
Device and method for measuring the concentration of a substance in a gaseous medium
Méthode et dispositif pour la mesure de la concentration d'une substance dans une médium gazeuse

(30) Priorität: 31.10.1996 DE 19643981
(43) Veröffentlichungstag der Anmeldung: 06.05.1998
(73) Patentinhaber: Testo GmbH & Co., 79853 Lenzkirch (DE)
(72) Erfinder: Münch, Reinhold, 79100 Freiburg (DE)
(74) Vertreter: Patentanwälte Westphal, Mussgnug & Partner

(56) Entgegenhaltungen:
- DE-A- 4 407 345
- US-A- 3 923 461
- US-A- 4 565 086
- PATENT ABSTRACTS OF JAPAN vol. 5, no. 196 (P-093), 12.Dezember 1981 & JP 56 118652 A (MITSUBISHI ELECTRIC CORP)

## Beschreibung

Die Erfindung betrifft ein Verfahren zum Ermitteln der Konzentration eines Stoffes in einem gasförmigen Medium gemäß Oberbegriff des Anspruchs 1 sowie eine hierzu geeignete Vorrichtung gemäß Oberbegriff des Anspruchs 11.

Chemische bzw. elektrochemische Sensoren zum Ermitteln der Konzentration von Stoffen in gasförmigen Medien besitzen einen sogenannten nominellen Meßbereich, in welchem die Messung mit definierter Genauigkeit möglich ist. Außerhalb des nominellen Meßbereichs arbeiten die Sensoren nur ungenau, auch besteht bei extremen Abweichungen vom nominellen Meßbereich die Gefahr von Beschädigungen.

Derartige Sensoren sind somit zunächst lediglich für solche Konzentrationsmessungen an gasförmigen Medien nur dann geeignet, wenn die zu erwartende Konzentration des zu messenden Stoffes innerhalb des nominellen Meßbereichs zu erwarten ist. Für eine Vielzahl von Anwendungen reicht der nominelle Meßbereich nicht aus, da die zu erwartenden Konzentrationswerte eine weitaus größere Bandbreite aufweisen können.

Zur Lösung dieser Problematik ist beispielsweise aus der DE 44 07 345 A1, von der die Erfindung ausgeht, bekannt, den chemischen Sensoren einen Meßgasstrom zuzuführen, der soweit verdünnt ist, daß die Konzentration des betreffenden Stoffes im nominellen Meßbereich des Sensors liegt. Durch Rückrechnen kann damit der aktuelle Wert der Konzentration des Stoffes im gasförmigen Medium bestimmt werden.

Konkret wird deshalb zunächst aus dem gasförmigen Medium ein Probengasstrom entnommen und in einer Mischeinrichtung mit einem Verdünnungasstrom, insbesondere einem aus der Umgebung gewonnenen Luftstrom, zu einem Meßgasstrom vermischt. Das Verdünnungsverhältnis kann hierbei durch Variation der Anteile von Probengasstrom und Verdünnungsgasstrom verändert und auf einem bestimmten Wert eingestellt werden. Der so gewonnene Meßgasstrom wird an den chemischen Sensor geleitet, welcher ein Detektionssignal als Maß für die Konzentration des Stoffes erzeugt. Sofern die detektierte Konzentration außerhalb des nominellen Meßbereichs liegt, wird durch Nachregulieren des Verdünnungsverhältnisses die Konzentration im Probengasstrom nachgeführt. Die Konzentration des Stoffes im gasförmigen Medium wird nunmehr durch Auswertung des Detektionssignals unter Berücksichtigung des Verdünnungsverhältnisses errechnet.

In diesem Zusammenhang kommt der möglichst exakten Kenntnis des Verdünnungsverhältnisses besondere Bedeutung zu, da dieses unmittelbar die Genauigkeit des Meßergebnisses bestimmt. Es wird zu diesem Zweck die Konzentration einer (weiteren) Stoffkomponente vor und nach der Verdünnung gemessen und daraus das Verdünnungsverhältnis bestimmt. Voraussetzung hierfür ist, daß diese (weitere) Stoffkomponente in einer der beiden Positionen, d. h. vor oder nach dem Vermischen bekannt und konstant ist. Eine der als bevorzugt wiedergegebenen Lösungsmöglichkeiten besteht darin, als Verdünnungsgasstrom einen Luftgasstrom zu verwenden, der aus der Umgebung abgezogen wird. Als bekannte und in konstanter Größe vorliegende Stoffkomponente vor dem Vermischen mit dem Probengasstrom kann beispielsweise Sauerstoff herangezogen werden. Aus dem Verhältnis der Sauerstoffkonzentration vor und nach dem Vermischen kann das Verdünnungsverhältnis zuverlässig bestimmt werden.

Nachteilig hierbei ist der vergleichsweise hohe Aufwand zur Ermittlung des Verdünnungsverhältnisses. So ist es erforderlich, einen zusätzlichen Sensor vorzusehen und insbesondere dessen Detektionssignal auszuwerten. Der damit verbundene Softwareaufwand ist nicht unerheblich, insbesondere als im Falle von dynamischen Vorgängen, d. h. Schwankungen der Konzentration des zu messenden Stoffes, das Verdünnungsverhältnis nachgeregelt werden muß.

Der Erfindung lag daher das Problem zugrunde, ein Verfahren und eine Vorrichtung zum Ermitteln der Konzentration eines Stoffes in einem gasförmigen Medium der eingangs genannten Art weiterzuentwickeln, bei dem die geschilderten Nachteile vermieden werden. Insbesondere soll die Einhaltung des nominellen Meßbereichs des chemischen Sensors auch bei Konzentrationswerten des Stoffes des gasförmigen Mediums außerhalb dieses nominellen Bereichs mit möglichst einfachen konstruktiven Mitteln und mit möglichst geringem Softwareaufwand realisiert werden.

Gelöst wird dieses Problem mit einem Verfahren, welches die Merkmale des Anspruchs 1 aufweist. Vorteilhafte Verfahrensvarianten sind durch die nachgeordneten Unteransprüche definiert.

Das Problem wird weiterhin durch eine für die Durchführung des Verfahrens geeignete Vorrichtung gelöst, welche durch die Merkmale des Anspruchs 11 angegeben ist. Vorteilhafte Ausführungsformen der Vorrichtung sind durch die nachgeordneten Unteransprüche angegeben.

Die Erfindung basiert auf der Idee, den Probengasstrom und den Verdünnungsgasstrom abwechselnd hintereinander zeitgetaktet zuzuführen und das Verdünnungsverhältnis aus dem Zeittaktverhältnis zu berechnen. Voraussetzung hierfür ist, daß der Probengasstrom und der Verdünnungsgasstrom mit jeweils definiertem Volumenstrom gefördert werden. Der Vorteil liegt darin, daß zur Bestimmung des Verdünnungsverhältnisses kein spezieller Sensor erforderlich ist. Das Zeittaktverhältnis läßt sich problemlos aus dem Quotienten derjenigen Zeitanteile bestimmen, die auf die Zuführung des Probengasstroms einerseits und des Verdünnungsgasstroms andererseits entfallen. Die Zeitmessung als solche ist trivial und kann in der Regel mit Hilfe eines von einer Mikroprozessoreinheit gesteuerten Timerbausteins erfolgen.

Besonders einfach gestaltet sich das Berechnen des Verdünnungsverhältnisses dann, wenn die Volumenströme des Probengasstromes und des Verdünnungsgasstromes übereinstimmenden Wert aufweisen. In diesem Fall entspricht das Zeittaktverhältnis bereits unmittelbar dem Verdünnungsverhältnis. Der Rechen- bzw. Softwareaufwand ist hierbei äußerst gering.

Vorteilhafterweise werden der Probengasstrom und der Verdünnungsgasstrom über ein Mischventil zugeführt, wobei ein besonders einfacher konstruktiver Aufbau durch die Verwendung eines 3/2-Wege-Ventils möglich wird, welches durch Hin- und Herschalten abwechselnd den Probengasstrom und den Volumengasstrom freigibt. Die Steuereinrichtung braucht in diesem Falle lediglich einen Umschaltimpuls zeitgetaktet vorgeben, um das gewünschte Verdünnungsverhältnis einzustellen. Ebenso ist es möglich, anstelle des 3/2-Wege-Ventils zwei parallelgeschaltete 2/2-Wege-Ventile zu verwenden.

Bevorzugt wird das Mischventil von einer zentralen Steuereinrichtung betätigt, die das Detektionssignal des chemischen Sensors auswertet und unmittelbar hieraus das erforderliche Zeittaktverhältnis bestimmt, welches zur Einhaltung des nominellen Meßbereichs erforderlich ist. Somit ist eine schnelle Ermittlung der Konzentration des Stoffes im gasförmigen Medium möglich.

Grundsätzlich ist damit das erfindungsgemäße Verfahren auch als dynamisches Meßverfahren auszulegen, bei dem das Verdünnungsverhältnis fortlaufend nachreguliert wird. Schwankungen der Konzentration des zu ermittelnden Stoffes werden durch kontinuierliche Anpassung des Verdünnungsverhältnisses soweit kompensiert, daß der Meßgasstrom stets eine Konzentration im nominellen Meßbereich des chemischen Sensors besitzt.

Im Extremfall ist es möglich, die Konzentration im Meßgasstrom praktisch konstant - und damit am optimalen Meßwert des Sensors - zu halten, so daß das Zeittaktverhältnis direkt proportional zur Ausgangskonzentration des Stoffes im gasförmigen Medium ist. Die Genauigkeit des Meßergebnisses hängt damit primär von der Genauigkeit der Zeitmessung ab, die in der Praxis keinerlei Probleme bereitet.

Als Verdünnungsgasstrom hat sich der Einsatz eines Luftstromes als vorteilhaft erwiesen, der unmittelbar aus der Umgebung gewonnen werden kann. In der Regel kann die Luft unmittelbar aus der Umgebung abgezogen und gegebenenfalls über ein Luftfilter unmittelbar der Mischeinrichtung zugeführt werden.

Ebenso ist es möglich, als Verdünnungsgasströme andere Gasströme, beispielsweise solche aus Flaschen, zu verwenden. Besonders geeignet erweist sich Stickstoff. Durch geeignete Maßnahmen, wie z. B. Pumpe mit Bypass, Druckregler mit Bypass, muß sichergestellt sein, daß der Verdünnungsgasstrom dem Mischventil drucklos zugeführt wird.

Eine erfindungsgemäße Weiterbildung sieht vor, daß mehrere chemische Sensoren zur Detektion mehrerer Stoffe des gasförmigen Mediums vorgesehen sind. Die Einstellung des Verdünnungsverhältnisses erfolgt in diesem Fall nach Maßgabe des Detektionssignals desjenigen chemischen Sensors, von dessen nominellen Meßbereich voraussichtlich zu Beginn der Messung am weitesten abgewichen wird. Hierdurch ist gewährleistet, daß der am stärksten gefährdete chemische Sensor sicher geschützt wird.

Bevorzugt kann ausgehend vom Detektionssignal einer oder mehrerer vorangegangener Messungen das voraussichtlich zu erwartende Detektionssignal der nunmehr anstehenden Messung bestimmt und hiernach das Verdünnungsverhältnis durch Gradientenbildung eingestellt werden. Durch diese Vorabschätzung des zu erwartenden Detektionssignals kann eine schnellere Nachregulierung in der Weise erfolgen, daß der Meßwert innerhalb des nominellen Meßbereichs liegt. In vielen Fällen kann damit auf das Nachregulieren verzichtet werden, da das aktuelle Detektionssignal bereits mit dem nominellen Meßbereich des Sensors korreliert.

Bei fortlaufenden Messungen kann das Verdünnungsverhältnis schrittweise verändert werden, nämlich dann, wenn der nominelle Meßbereich überschritten wird. Andererseits besteht die Möglichkeit einer kontinuierlichen, stufenlosen Veränderung des Verdünnungsverhältnisses, so daß der Meßwert in einem relativ engen Intervall des nominellen Meßbereichs bzw. im Meßwertoptimum gehalten werden kann.

Das erfindungsgemäß Verfahren einer dynamischen Verdünnung eines von einem gasförmigen Medium gezogenen Probenstroms zur Einhaltung des nominellen Meßbereichs chemischer Sensoren verwendet elektrische Detektionssignale dieser Sensoren, um festzustellen, ob der nominelle Meßbereich eingehalten wird. Liegt das Detektionssignal außerhalb des nominellen Meßbereichs (als Teilbereich eines gesamten Meßbereichs), so wird das Verdünnungsverhältnis verändert. Das Verdünnungsverhältnis wird hierbei über durch zeitgetaktetes und abwechselnd aufeinanderfolgendes Zuführen von Probengasstrom und Verdünnungsgasstrom bewerkstelligt.

Eine zur Durchführung des Verfahrens geeignete Vorrichtung weist folgende Hauptkomponenten auf:
a) Eine Einrichtung zum Entnehmen des Probengasstroms,
b) eine Verdünnungseinrichtung zum Verdünnen des Probengasstroms mit einem Verdünnungsgasstrom, in der der Probengasstrom und der Verdünnungsgasstrom über ein Mischventil zeitgetaktet abwechselnd einer Mischeinrichtung zugeführt und dort zu einem Meßgasstrom vermischt werden,
c) wenigstens einem chemischen Sensor zum Erzeugen eines Detektionssignals als Maß für die Konzentration des Stoffes im Meßgasstrom, und
d) eine Steuereinrichtung, um nach Maßgabe der detektierten Konzentration das Verdünnungsverhältnis durch Vorgabe des Zeittaktes derart nachzuregulieren, daß die Konzentration des Stoffes im Meßgasstrom im nominellen Meßbereich des chemischen Sensors gehalten wird.

Diese Vorrichtung zeichnet sich durch einen konstruktiv einfachen Aufbau aus, auch gestaltet sich die Einstellung des Verdünnungsverhältnisses bzw. deren Nachführung äußerst einfach.

Vorteilhafterweise ist die Mischeinrichtung als Mischkammer mit integrierten Turbulenzerzeugern ausgeführt, um eine möglichst intensive homogene Durchmischung des Probengasstroms und des Verdünnungsgasstroms sicherzustellen. Dies ist im Zusammenhang mit vorliegender Erfindung besonders von Bedeutung, da die Teilgasströme abwechselnd der Mischkammer zugeführt werden. Als Turbulenzerzeuger eignen sich beispielsweise Düsen, Kapillaren oder sogenannte statische Mischer, die durch Erzeugung starker Turbulenzen eine innige Vermischung auf relativ kleinem Volumen ermöglichen.

Bevorzugt ist ein Bypass zur Umgehung der Verdünnungseinrichtung vorgesehen, über den der gezogene Probengasstrom unmittelbar den chemischen Sensoren zugeführt wird. Eine solche Variante bietet sich dann an, wenn aufgrund der zu erwartenden Konzentrationswerte die chemischen Sensoren im nominellen Meßbereich betrieben werden können und nur im Einzelfall eine Verdünnung wegen Meßwertüberschreitung nötig wird.

Von Vorteil kann unmittelbar vor dem Mischventil eine Vorkammer vorgeschaltet sein, die als eine Art Puffer dient und Schwankungen im Zuführbereich des Probengasstroms ausgleichen kann.

Schließlich ist eine Förderpumpe für den Meßgasstrom von Vorteil, welche der Mischkammer nachgeschaltet ist. In Abhängigkeit der augenblicklichen Stellung des Mischventils fördert diese entweder Probengas oder Verdünnungsgas. Die gewählte Anordnung hinter der Mischkammer stellt sicher, daß der Meßgasstrom in gleichmäßiger Durchmischung von der Pumpe angesaugt und den Sensoren zugeführt wird.

Die Erfindung wird nachstehend näher anhand der in den Figuren dargestellten Ausführungsbeispiele erläutert. Es zeigen:
- Figur 1: Prinzipschaltbild zur Durchführung des Verfahrens und
- Figur 2: Prinzipschaltbild einer hierzu geeigneten Vorrichtung.

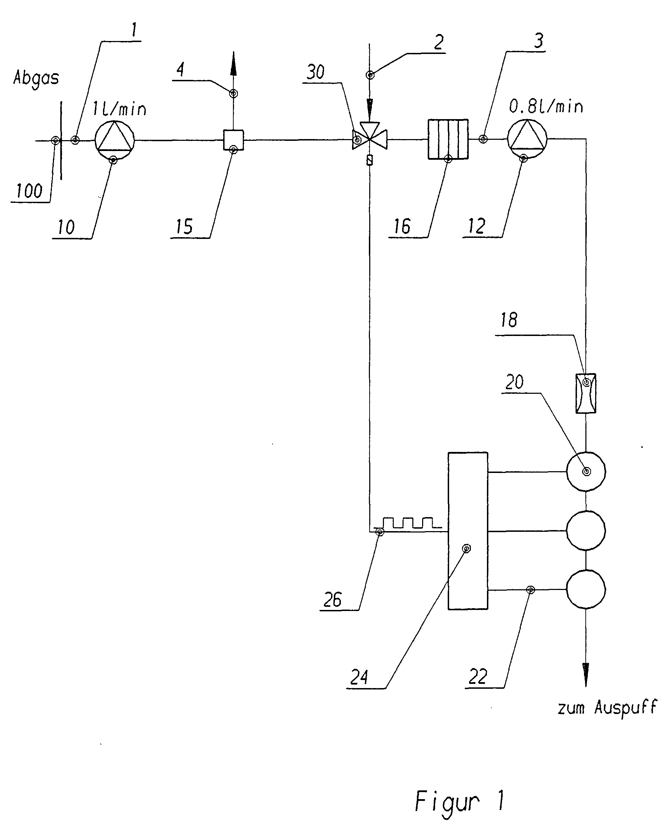

Die grundsätzliche Verfahrensführung ergibt sich aus Figur 1. Ein zu untersuchendes gasförmiges Medium 100, beispielsweise Abgas einer Feuerungsanlage, soll hinsichtlich des Gehalts (Konzentration) bestimmter Stoffe untersucht werden. Hierfür sind chemische Sensoren 20 vorgesehen, deren Detektionssignale 22 einer zentralen Steuereinrichtung 24 zugeführt werden, in welcher diese ausgewertet und beispielsweise angezeigt, gespeichert oder weiterverarbeitet werden. Die chemischen Sensoren 20 haben jeweils einen spezifischen, nominellen Meßbereich für die Konzentration des zu untersuchenden Stoffes, der mit einem nominellen Wertebereich des Detektionssignals 22 korreliert.

Dem gasförmigen Medium 100 wird ein Probengasstrom 1 entnommen. Hierzu ist eine Förderpunmpe 10 vorhanden, die den Probengasstrom 1 kontinuierlich und mit einem bestimmten Volumenstrom fördert, welcher unter allen Betriebszuständen stets konstant ist. Der Probengasstrom 1 wird über ein Mischventil 30 einer Mischkammer 16 zugeführt. Das Mischventil 30 ist ein getaktetes 3/2-Wege-Ventil, welches von der Steuereinrichtung 24 durch ein Steuersignal 26 betätigt wird.

Über das Mischventil 30 wird weiterhin ein Verdünnungsgasstrom 2 der Mischkammer 16 zugeführt, in der durch hier nicht näher dargestellte Turbulenzerzeuger eine intensive Vermischung mit dem Probengasstrom 1 zu einem Meßgasstrom 3 erfolgt. Der Meßgasstrom 3 wird mit der Förderpumpe 12 mit einem definierten und konstant gehaltenen Volumenstrom über eine Kapillare 18 den chemischen Sensoren 20 zugeführt. Die chemischen Sensoren 20 erfassen damit die Konzentration der betreffenden Stoffe im (verdünnten) Meßgasstrom 3, die in Abhängigkeit des Verdünnungsverhältnisses eine Rückrechnung auf die im gasförmigen Medium 100 vorhandene (wahre) Konzentration rückgerechnet werden müssen.

Das Mischventil 30 schaltet getaktet zwischen dem Probengasstrom 1 und dem Verdünnungsgasstrom 2 abwechselnd hin und her, so daß im (hier nicht näher bezeichneten) Leitungsabschnitt zwischen dem Mischventil 30 und der Mischkammer 16 jeweils aufeinanderfolgend Teilvolumina ("Pakete") des Probengasstroms 1 und des Verdünnungsgasstroms 2 vorhanden sind. Da das Mischventil 30 lediglich zwei Schaltzustände kennt, wird entweder der Pfad für den Probengasstrom 1 oder derjenige für den Verdünnungsgasstrom 2 freigegeben. Solange das Mischventil 30 den Pfad für den Probengasstrom 1 freigibt, ist der Pfad für den Verdünnungsgasstrom 2 geschlossen. Beim Umschalten des Mischventils 30 wird der Pfad für den Probengasstrom 1 geschlossen und gleichzeitig derjenige für den Verdünnungsgasstrom 2 freigegeben. Der Verdünnungsgasstrom 2 entsteht dadurch, daß die Förderpumpe 12 über die Mischkammer 16 und das Mischventil 30 Luft aus der Umgebung ansaugt.

Da während der Dauer dieses Schaltzustandes die Förderpumpe 10 weiterhin Probengas fördert, ist in Strömungsrichtung betrachtet vor dem Mischventil 30 eine Verzweigung 14 vorhanden, über die der Probengasstrom 1 als Bypassstrom 4 beispielsweise zum gasförmigen Medium 100 im Abströmbereich (Auspuff) zurückgeleitet wird. In diesem Fall entspricht der Volumenstrom des Probengasstroms 1 dem Volumenstroms des Bypassstroms 4.

Sobald das Mischventil 30 erneut die Leitung für den Probengasstrom 1 freischaltet wird der Probengasstrom der Mischkammer 16 zugeführt, und zwar mit einem Volumenstrom, der im durch die Förderpumpe 12 vorgegeben ist. Da dieser Volumenstrom kleiner ist als der durch die Förderpumpe 10 geförderte Probengasstrom 1 entweicht ein Teil des Probengases weiterhin in Form eines Bypassstromes 4.

Aus dem Vorstehenden ergibt sich, daß das Verdünnungsverhältnis, d. h. das Verhältnis aus dem Volumenstrom des Probegasstroms 1 zum Volumenstrom des Verdünnungsgasstroms 2 durch Variation des Zeittaktverhältnisses eingestellt werden kann, mit welchem das Mischventil 30 hin- und hergeschaltet wird. Da die hierfür erforderlichen Steuersignale 26 von der zentralen Steuereinrichtung 24 erzeugt werden, kann auf einfache Art und Weise durch Auswertung der Detektionssignale 22 sofort nachgeregelt werden, wenn die Detektionssignale 22 nicht innerhalb des vorgesehenen Wertebereichs liegen sollten. Durch diese einfache Maßnahme ist sichergestellt, daß durch Variation des Verdünnungsverhältnisses die chemischen Sensoren stets in ihrem nominellen Meßbereich betrieben werden. Die Konzeption erlaubt insbesondere auch eine fortwährende (dynamische) Erfassung der Konzentration der Stoffe, auch wenn die Meßwerte sehr starken zeitlichen Schwankungen unterworfen sein sollten.

Sofern - wie im vorliegenden Ausführungsbeispiel realisiert - die Volumenströme des Probengasstroms 1 und Verdünnungsgasstroms 2 in Übereinstimmung sind, kann unmittelbar aus dem Zeittaktverhältnis und dem im Meßgasstrom 3 ermittelten Konzentrationswert des Stoffes auf den im gasförmigen Medium 100 vorliegenden (wahren) Konzentrationswert umgerechnet werden. Wird beispielsweise das Mischventil 30 im Verhältnis 1/10 Sekunde zu 9/10 Sekunde zwischen dem Probengasstrom 1 und dem Verdünnungsgasstrom 2 hin und hergeschaltet, ergibt dies einen Meßgasstrom 3 mit einem Verdünnungsverhältnis von 1 zu 10. Der durch die chemischen Sensoren 20 detektierten Werte der Konzentration brauchen somit lediglich mit dem Faktor 10 multipliziert werden, um die tatsächlich vorherrschende Konzentration zu bestimmen. Der effektive Meßbereich wurde in diesem Fall um den Faktor 10 erhöht.

Die Steuereinrichtung 24 kann beispielsweise derart programmiert sein, daß das Zeittaktverhältnis in vorgegebenen Stufen immer dann verändert wird, wenn das aktuelle Detektionssignal 22 den vorgegenbenen Wertebereich zu verlassen droht. Zweckmäßig in diesem Zusammenhang ist jeweils eine Verdoppelung des Zeittaktverhältnisses, so daß sich Verdünnungsschritte in dualer Reihenfolge ergeben.

Ebenso ist es möglich, die Veränderung des Verdünnungsverhältnisses stufenlos durchzuführen, wodurch es letztendlich möglich ist, den Meßwert stets auf einem bestimmten, vorgegebenen Optimalwert zu halten, um die aktuelle (wahre) Konzentration des Stoffes ausschließlich mit dem (sich kontinuierlich verändernden) Verdünnungsverhältnis zu korrelieren. Hierzu ist allerdings ein vergleichsweise hoher rechnerischer Aufwand nötig, um Totzeiten im Gasweg und Verzögerungszeiten der Sensoren zu berücksichtigen.

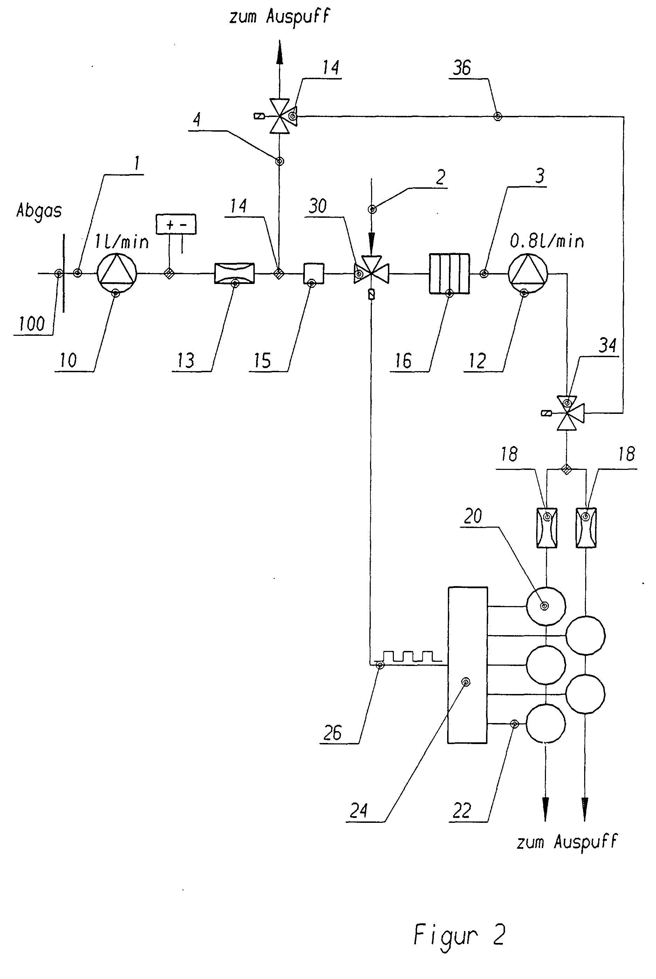

Die in Figur 2 dargestellte Konfiguration baut auf der Ausgangskonfiguration gemäß Figur 1 unmittelbar auf. Neben der höheren Zahl von chemischen Sensoren 20, die in zwei parallel geschaltete Pfade aufgespalten sind, fällt insbesondere eine Bypassleitung 36 auf, die sämtliche Komponenten umgeht, die die Verdünnung des Probengasstroms 1 bewerkstelligen. Die Bypassleitung 36 zweigt an der Verzweigung 14 ab und wird hinter der Förderpumpe 12 und vor den Kapillaren 18 rückgeführt. Sie ermöglicht damit weiterhin den sogenannten Normalbetrieb, bei dem der Probengasstrom 1 unverdünnt den chemischen Sensoren 20 zugeführt wird. Zusätzlich ist in die Bypassleitung 36 ein Ventil 32 eingeschaltet, welches als 3/2-Wege-Ventil ausgeführt ist und je nach Schaltstellung die Bypassleitung 36 durchschaltet oder diese unter Freigabe eines zum Auspuff führenden Pfades unterbricht.

Weiterhin ist ein Ventil 34 am Rückführpunkt der Bypassleitung 36 vorgesehen, welches alternativ den Pfad über die Bypassleitung 36 oder den Pfad für den Meßgasstrom 3 freigibt.

Im Normalbetrieb sind die Ventile 32, 34 (und auch das Mischventil 30) in Ruhestellung, so daß der Probengasstrom 1 den Pfad über die Bypassleitung 36 nimmt. Der Probengasstrom 1 wird durch die Förderpumpe 10 aufrecht erhalten. Die Förderpumpe 12 ist abgeschaltet.

Für den Verdünnungsbetrieb sind die Ventile 32, 34 durchgeschaltet (bestromt). Die Förderpumpe 10 fördert Probengas in eine als Puffer wirkende Vorkammer 15, und von dort über das getaktete Mischventil 30 in die Mischkammer 16. Überschüssiges Probengas kann nach wie vor über die Verzweigung 14 und das durchgeschaltete Ventil 32 zum Auspuff abgeblasen werden. Weiterhin wird durch die Förderpumpe 12 Verdünnungsgasstrom über das getaktete Mischventil 30 angesaugt und ebenfalls der Mischkammer 16 zugeführt. Diese Betriebsart stimmt damit mit der eingangs beschriebenen Verfahrensführung überein.

Der Vollständigkeit halber sei erwähnt, daß sich der sogenannte Normalbetrieb, bei dem der Probengasstrom 1 nicht verdünnt wird, grundsätzlich auch mit der Konfiguration gemäß Figur 1 realisieren läßt. Hierzu ist das Mischventil 30 konstant auf den Pfad des Probengasstroms 1 durchgeschaltet.
Aufgrund der hohen Meßgenauigkeit eignet sich die Erfindung nicht nur zur Messung der Zusammensetzung eines hochkonzentrierten Abgases durch Verdünnung, sondern kann auch dazu eingesetzt werden, hochkonzentriertes Prüfgas bekannter Zusammensetzung definiert zu verdünnen, um hierdurch die Kalibrierung von Meßeinrichtungen anhand einer Meßreihe unterschiedlicher Konzentration durchzuführen.

### Bezugszeichenliste

- 1: Probengasstrom
- 2: Verdünnungsgasstrom
- 3: Meßgasstrom
- 4: Bypassstrom

- 10: Förderpumpe
- 12: Förderpumpe
- 13: Kapillare
- 14: Verzweigung
- 15: Vorkammer
- 16: Mischkammer
- 18: Kapillare

- 20: chemischer Sensor
- 22: Detektionssiganl
- 24: Steuereinrichtung
- 26: Steuersignal

- 30: Mischventil
- 32: Ventil
- 34: Ventil
- 36: Bypassleitung

- 100: gasförmiges Medium

## Patentansprüche

1. Verfahren zum Ermitteln der Konzentration eines Stoffes in einem gasförmigen Medium mit einem chemischen Sensor, der einen nominellen Meßbereich für die Konzentration des Stoffes aufweist, durch
a) Entnehmen eines Probengasstroms aus dem gasförmigen Medium,
b) Vermischen des Probengasstroms mit einem Verdünnungsgasstrom in einer Mischeinrichtung zu einem Meßgasstrom in einem einstellbaren Verdünnungsverhältnis,
c) Zuführen des Meßgasstroms an den chemischen Sensor zum Erzeugen eines Detektionssignals,'
d) Nachregulieren des Vedünnungsverhältnisses derart, daß die vom chemischen Sensor detektierte Konzentration des Stoffes im nominellen Meßbereich gehalten wird,
dadurch gekennzeichnet,
daß der Probengasstrom (1) und der Verdünnungsgasstrom (2) jeweils abwechselnd hintereinander mit definiertem Volumenstrom zeitgetaktet der Mischeinrichtung (16) zugeführt worden, und
daß das Verdünnungsverhältnis auf der Basis des Zeittaktverhältnisses berechnet wird.

2. Verfahren nach Anspruch 1, dadurch gekennzeichnet, daß die Volumenströme des Probengasstromes (1) und des Verdünnungsgasstromes (2) einen übereinstimmenden Wert aufweisen.

3. Verfahren nach Anspruch 1 oder 2, dadurch gekennzeichnet, daß der Probengasstrom (1) und der Verdünnungsgasstrom (2) über ein vor der Mischeinrichtung (16) angeordnetes Mischventil, vorzugsweise in Form eines 3/2-Wege-Ventils (30), zugeführt werden, das abwechselnd den Probengasstrom (1) und den Verdünnungsgasstrom (2) freigibt.

4. Verfahren nach Anspruch 3, dadurch gekennzeichnet, daß das Mischventil (30) von einer zentralen Steuereinrichtung (24) betätigt wird, die das Zeittaktverhältnis in Abhängigkeit des Detektionssignals (22) vorgibt.

5. Verfahren nach einem der vorhergehenden Ansprüche, dadurch gekennzeichnet, daß das Verdünnungsverhältnis fortlaufend nachreguliert wird.

6. Verfahren nach einem der vorhergehenden Ansprüche, dadurch gekennzeichnet, daß als Vedünnungsgasstrom (2) ein aus der Umgebung gewonnener Luftstrom oder ein aus Flaschen oder dergleichen gewonnener Gasstrom verwendet wird.

7. Verfahren nach einem der vorhergehenden Ansprüche, dadurch gekennzeichnet, daß zu Beginn das Verdünnungsverhältnis so groß eingestellt wird, daß der chemische Sensor (20) sicher in seinem nominellen Meßbereich arbeitet.

8. Verfahren nach einem der vorhergehenden Ansprüche, dadurch gekennzeichnet, daß mehrere chemische Sensoren (20) zur Detektion mehrerer unterschiedlicher Stoffe vorgesehen sind und eine Regelung des Verdünnungsverhältnisses nach Maßgabe des Detektionssignales (22) desjenigen chemischen Sensors (20) erfolgt, von dessen nominellen Meßbereich zu Beginn am weitesten abgewichen wird.

9. Verfahren nach einem der vorhergehenden Ansprüche, dadurch gekennzeichnet, daß ausgehend vom Detektionssignal (22) des chemischen Sensors (20) einer vorangegangenen Messung das voraussichtlich zu erwartende Detektionssignal (22) der folgenden Messung bestimmt und davon ausgehend das Verdünnungsverhältnis durch Gradientenbildung eingestellt wird.

10. Verfahren nach einem der vorhergehenden Ansprüche, dadurch gekennzeichnet, daß das Detektionssignal (22) des chemischen Sensors (20) in einem vorbestimmten Intervall des nominellen Meßbereiches durch schrittweise oder stufenlose Veränderung des Verdünnungsverhältnisses gehalten wird.

11. Vorrichtung zur Durchführung des Verfahrens nach einem der Ansprüche 1 bis 10, gekennzeichnet durch
a) eine Einrichtung zum Entnehmen des Probengasstromes (1)
b) eine Verdünnungseinrichtung zum Verdünnen des Probengasstroms (1) mit einem Verdünnungsgasstrom (2), in der der Probengasstrom (1) und der Verdünnungsgasstrom (2) über ein Mischventil (30), vorzugsweise in Form eines 3/2-Wege-Ventils, zeitgetaktet abwechselnd einer Mischeinrichtung (16) zugeführt und dort zu einem Meßgasstrom (3) vermischt werden,
c) wenigstens einen chemischen Sensor (20) zum Erzeugen eines Detektionssignals (22) als Maß für die Konzentration des Stoffes im Meßgasstrom (3), und
d) eine Steuereinrichtung (24), um nach Maßgabe der detektierten Konzentration das Verdünnungsverhältnis durch Vorgabe des Zeittaktes derart nachzuregulieren, daß die Konzentration des Stoffes im Meßgasstrom (3) im nominellen Meßbereich des chemischen Sensors (20) gehalten wird.

12. Vorrichtung nach Anspruch 11, dadurch gekennzeichnet, daß die Mischeinrichtung (24) als Mischkammer mit integrierten Turbulenzerzeugern, wie beispielsweise Düsen, Kapillaren oder statischen Mischern, ausgeführt ist.

13. Vorrichtung nach Anspruch 11 oder 12 gekennzeichnet durch einen schaltbaren Bypass zur Umgehung der Verdünnungseinrichtung.

14. Vorrichtung nach einem der Ansprüche 11 bis 13, gekennzeichnet durch eine dem Mischventil (30) vorgeschaltete Vorkammer (15).

15. Vorrichtung nach einem der Ansprüche 11 bis 14, gekennzeichnet durch eine der Mischkammer (16) nachgeschaltete Förderpumpe (12) für den Meßgasstrom.

## Claims

1. Method for measuring the concentration of a substance in a gaseous medium using a chemical sensor, which has a nominal measurement range for the concentration of the substance, by
a) extracting a sample gas flow from the gaseous medium,
b) mixing the sample gas flow with a dilution gas flow in a mixing device to form a measurement gas flow at a settable dilution ratio,
c) delivering the measurement gas flow to the chemical sensor in order to produce a detection signal,
d) re-adjusting the dilution ratio in such a way that the substance concentration detected by the chemical sensor is kept in the nominal measurement range,
characterised in that
the sample gas flow (1) and the dilution gas flow (2) are each delivered alternately for timed intervals, in succession, to the mixing device (16) at a defined volumetric flow rate, and
the dilution ratio is calculated on the basis of the timed-interval ratio.

2. Method according to Claim 1, characterised in that the volumetric flow rates of the sample gas flow (1) and of the dilution gas flow (2) have a matching value.

3. Method according to Claim 1 or 2, characterised in that the sample gas flow (1) and the dilution gas flow (2) are delivered through a mixing valve arranged upstream of the mixing device (16), preferably in the form of a 3/2 way valve (30), which lets through the sample gas flow (1) and the dilution gas flow (2) alternately.

4. Method according to Claim 3, characterised in that the mixing valve (30) is actuated by a central control device (24), which specifies the timed-interval ratio as a function of the detection signal (22).

5. Method according to one of the preceding claims, characterised in that the dilution ratio is re-adjusted constantly.

6. Method according to one of the preceding claims, characterised in that an air flow obtained from the surroundings, or a gas flow obtained from cylinders or the like, is used as the dilution gas flow (2).

7. Method according to one of the preceding claims, characterised in that the dilution ratio is initially set to a value which ensures that the chemical sensor (20) operates in its nominal measurement range.

8. Method according to one of the preceding claims, characterised in that a plurality of chemical sensors (20) are provided in order to detect a plurality of different substances, and adjustment of the dilution ratio is carried out in accordance with the detection signal (22) of that chemical sensor (20) from whose nominal measurement range there is initially the greatest deviation.

9. Method according to one of the preceding claims, characterised in that the detection signal (22) of the chemical sensor (20) from a previous measurement is used to determine the likely expected detection signal (22) for the following measurement, and this is used to set up the dilution ratio by taking the gradient.

10. Method according to one of the preceding claims, characterised in that the detection signal (22) of the chemical sensor (20) is kept in a predetermined interval of the nominal measurement range by incremental or continuous modification of the dilution ratio.

11. Device for carrying out the method according to one of Claims 1 to 10, characterised by
a) a device for extracting the sample gas flow (1),
b) a dilution device for diluting the sample gas flow (1) with a dilution gas flow (2), in which the sample gas flow (1) and the dilution gas flow (2) are delivered alternately for timed intervals, through a mixing valve (30), preferably in the form of a 3/2 way valve, to a mixing device (16) where they are mixed to form a measurement gas flow (3),
c) at least one chemical sensor (20) for producing a detection signal (22) as a measure of the concentration of the substance in the measurement gas flow (3), and
d) a control device (24), for re-adjusting the dilution ratio in accordance with the detected concentration, by specifying the timing cycle in such a way that the concentration of the substance in the measurement gas flow (3) is kept in the nominal measurement range of the chemical sensor (20).

12. Device according to Claim 11, characterised in that the mixing device (24) is designed as a mixing chamber with integrated turbulence generators such as nozzles, capillaries or static mixers, for example.

13. Device according to Claim 11 or 12, characterised by a switchable bypass for circumventing the dilution device.

14. Device according to one of Claims 11 to 13, characterised by an ante-chamber (15) connected upstream of the mixing valve (30).

15. Device according to one of Claims 11 to 14, characterised by a feed pump (12) for the measurement gas flow, connected downstream of the mixing chamber (16).

## Revendications

1. Procédé pour déterminer la concentration d'une substance dans un milieu gazeux à l'aide d'un capteur chimique ayant une plage de mesure nominale pour la concentration de la matière, procédé selon lequel :
a) on prélève un flux de gaz échantillon dans le milieu gazeux,
b) on mélange le flux de gaz échantillon à un flux de gaz de dilution dans une installation de mélange pour former un flux de gaz de mesure selon un coefficient de dilution réglable,
c) on fournit le flux de gaz de mesure au capteur chimique pour donner un signal de détection,
d) on asservit le coefficient de dilution pour que la concentration de la substance détectée par le capteur chimique reste dans la plage nominale,
caractérisé en ce qu'
• on fournit le flux de gaz échantillon (1) et le flux de gaz de dilution (2), chaque fois successivement en alternance selon un débit volumique défini, de manière cadencée à l'installation de mélange (16),
• on calcule le coefficient de dilution à partir du rapport de cadence.

2. Procédé selon la revendication 1,
caractérisé en ce que
les débits volumiques du flux de gaz échantillon (1) et du flux de gaz de dilution (2) correspondent à une valeur concordante.

3. Procédé selon la revendication 1 ou 2,
caractérisé en ce que
le flux de gaz échantillon (1) et le flux de gaz de dilution (2) sont fournis par une vanne mélangeuse installée en amont de l'installation de mélange (16), de préférence sous la forme d'un distributeur à 3/2 voies (30) qui libère en alternance le flux de gaz échantillon (1) et le flux de gaz de dilution (2).

4. Procédé selon la revendication 3,
caractérisé en ce qu'
on actionne la vanne mélangeuse (30) à l'aide d'une installation de commande centrale (24) qui prédéfinit le rapport de cadence en fonction du signal de détection (22).

5. Procédé selon l'une quelconque des revendications précédentes,
caractérisé en ce qu'
on asservit en permanence le coefficient de dilution.

6. Procédé selon l'une quelconque des revendications précédentes,
caractérisé en ce qu'
on utilise comme flux de gaz de dilution (2), un flux d'air pris dans l'environnement ou un flux de gaz prélevé dans une bouteille.

7. Procédé selon l'une quelconque des revendications précédentes,
caractérisé en ce qu'
au début, on règle le coefficient de dilution à un niveau suffisamment grand pour que le capteur chimique (20) travaille avec certitude dans sa plage de mesure nominale.

8. Procédé selon l'une quelconque des revendications précédentes,
caractérisé en ce qu'
on utilise plusieurs capteurs chimiques (20) pour détecter plusieurs substances différentes et on régule le coefficient de dilution à partir du signal de détection (22) de celui des capteurs chimiques (20) pour lequel la déviation par rapport à la plage de mesure nominale est initialement la plus grande.

9. Procédé selon l'une quelconque des revendications précédentes,
caractérisé en ce que
partant du signal de détection (22) du capteur chimique (20) d'une mesure précédente, on définit le signal de détection (22) prévisible pour la mesure suivante et partant delà on règle le coefficient de dilution en formant un gradient.

10. Procédé selon l'une quelconque des revendications précédentes,
caractérisé en ce qu'
on maintient le signal de détection (22) du capteur chimique (20) dans un intervalle prédéterminé de la plage de mesure nominale en modifiant pas à pas ou en continu le coefficient de dilution.

11. Dispositif pour la mise en oeuvre du procédé selon l'une quelconque des revendications 1 à 10,
caractérisé par
a) une installation pour prélever le flux de gaz échantillon (1),
b) une installation de dilution pour diluer le flux de gaz échantillon (1) avec un flux de gaz de dilution (2) qui reçoit le flux de gaz échantillon (1) et le flux de gaz de dilution (2) par une vanne mélangeuse (30), de préférence un distributeur à 3/2 voies, de façon cadencée, en alternance dans une installation de mesure (16) pour y être mélangé et former un flux de gaz de mesure (3),
c) au moins un capteur chimique (20) pour générer un signal de détection (22) comme mesure de la concentration de la substance dans le flux de gaz de mesure (3), et
d) une installation de commande (24) pour qu'en fonction de la concentration détectée, le coefficient de dilution soit asservi en prédéfinissant le rapport de cadence, de sorte que la concentration de la substance dans le flux de gaz de mesure (3) reste dans la plage de mesure nominale du capteur chimique (20).

12. Dispositif selon la revendication 11,
caractérisé en ce que
l'installation de mesure (24) est une chambre de mélange intégrant des générateurs de turbulence tels que des buses, tubes capillaires ou mélangeurs statiques.

13. Dispositif selon la revendication 11 ou 12,
caractérisé par
une dérivation commutable pour contourner l'installation de dilution.

14. Dispositif selon l'une quelconque des revendications 11 à 13,
caractérisé par
une antichambre (15) en amont de la vanne mélangeuse (30).

15. Dispositif selon l'une quelconque des revendications 11 à 14,
caractérisé par
une pompe d'alimentation (12) en aval de la chambre de mélange (16) pour le flux de gaz de mesure.
